# EUROPEAN PATENT APPLICATION

(11) **EP 4 700 132 A1**
(43) Date of publication of application: **25.02.2026**
(21) Application number: 25197409.3
(22) Date of filing: 21.08.2025
(51) Int. Cl.: C12Q 1/6806

(54) **DETECTION METHOD FOR TARGET ECDNA AND DETECTION KIT FOR TARGET ECDNA**

(30) Priority: 21.08.2024 JP 2024139562
(71) Applicant: Canon Medical Systems Corporation, Tochigi (JP)
(72) Inventor: FUJITA, Keigo, Otawara-shi (JP); SATO, Suguru, Otawara-shi (JP); SATAKE, Hiroto, Otawara-shi (JP)
(74) Representative: Marks & Clerk LLP

(57) **Abstract**

According to an embodiment, there is provided a method of detecting a target ecDNA in a sample containing chromosomal DNA and ecDNA, where the method includes (a) a step of subjecting the DNA in the sample to a treatment of sequence-specifically cleaving a third sequence present between a first sequence and a second sequence in a target nucleotide sequence included in the target ecDNA, (b) a step of separating the sample after the step (a) into a plurality of compartments; and (c) a step of detecting a compartment containing DNA having both the first sequence and the second sequence in the plurality of compartments after the step (b).

## Description

### BACKGROUND OF THE INVENTION

### Field of the Invention

An embodiment disclosed in the present specification and the drawing relates to a detection method for a target ecDNA and a detection kit for a target ecDNA.

Extrachromosomal DNA (ecDNA) is circular DNA derived from chromosomal DNA, which is present in cells outside the chromosomes. ecDNA is considered to cause amplification of cancer genes, acquisition of resistance to anti-cancer agents, and tumor heterogeneity, thereby being associated with poor prognosis in cancer patients.

As a method of detecting ecDNA, a method using a next generation sequence, a method using fluorescence in situ hybridization (FISH), and the like are known. In addition, a method of detecting ecDNA using a chromatin interaction, and a method of isolating ecDNA by pulse field gel electrophoresis are known.

### SUMMARY OF THE INVENTION

However, the detection method for the ecDNA in the related art has problems in terms of the time until the results are obtained, cost, complexity of analysis, complicated analysis, detection sensitivity, and the like.

An object to be achieved by the embodiment disclosed in the present specification and the drawings is to shorten the time taken until the results are obtained in a specimen in which the chromosomal DNA and the ecDNA are mixedly present, and to detect the ecDNA at a low cost. However, the object to be achieved according to the embodiment disclosed in the present specification and the drawing is not limited to the above object. The object corresponding to each effect according to each configuration shown in an embodiment which is described later may be positioned as another object.

According to an embodiment, a method of detecting a target ecDNA including a target nucleotide sequence in a specimen containing DNA includes (a) a step of subjecting the DNA in the specimen to a treatment of sequence-specifically cleaving a third sequence present between a first sequence and a second sequence in the target nucleotide sequence included in the target ecDNA; (b) a step of separating the specimen into a plurality of compartments after the step (a); and (c) a step of detecting a compartment containing DNA having both the first sequence and the second sequence in the plurality of compartments after the step (b).

In the preceding embodiment, the step (a) may be carried out by using a guide RNA and an RNA-induced nuclease that targets the third sequence.

In any one of the preceding embodiments, the step (c) may include carrying out a nucleic acid amplification reaction for a region containing the first sequence and a nucleic acid amplification reaction for a region containing the second sequence.

In any one of the preceding embodiments, in the step (c), a compartment containing DNA that has both the first sequence and the second sequence but does not have the third sequence which is uncleaved may be detected.

In any one of the preceding embodiments, the step (c) may include carrying out a nucleic acid amplification reaction for a region containing the first sequence, a nucleic acid amplification reaction for a region containing the second sequence, and a nucleic acid amplification reaction for a region containing the third sequence.

In any one of the preceding embodiments, the step (b) may be carried out so that one molecule or fewer of DNA is distributed in one compartment.

In any one of the preceding embodiments, the target nucleotide sequence may be a nucleotide sequence included in a cancer gene.

In any one of the preceding embodiments, the step (b) and the step (c) may be carried out by digital PCR.

In any one of the preceding embodiments, in the step (c), a first fluorescent dye may be used to detect a compartment having the first sequence, and a second fluorescent dye is used to detect a compartment having the second sequence.

In any one of the preceding embodiments, in the step (c), a third fluorescent dye may be used to detect a compartment having the third sequence.

In any one of the preceding embodiments, the method may further include (d) a step of excluding a compartment containing at least two molecules of DNAs from the compartment detected in the step (c), where the two molecules of DNAs are a DNA including the first sequence and a DNA including the second sequence.

In any one of the preceding embodiments, the step (d) may be carried out using the following numerical expressions (i) and (ii), $\frac{\left\{p+N\left(A\right)\right\} \left\{p+N\left(C\right)\right\}}{N \left(U\right)} = p$ $q = N \left(E\right) - p$ wherein, p is the number of compartments which are assumed to contain at least two molecules of DNAs, which are a DNA having the first sequence and a DNA having the second sequence, N(A) is the number of compartments in which only the first sequence is detected, N(C) is the number of compartments in which only the second sequence is detected, N(U) is the number of all compartments in the step (b), q is the number of compartments containing the target ecDNA, and N(E) is the number of compartments in which the first sequence and the second sequence have been detected but the third sequence has not been detected.

According to an embodiment, a kit for detecting a target ecDNA including a target nucleotide sequence includes (a) a cleaving reagent for sequence-specifically cleaving a third sequence present between a first sequence and a second sequence in the target nucleotide sequence, (b) a detection reagent for the first sequence; and (c) a detection reagent for the second sequence.

In the preceding embodiment, the cleaving reagent may contain a guide RNA and an RNA-induced nuclease that targets the third sequence.

In any one of the preceding embodiments, the kit may further include (d) a detection reagent for the third sequence.

### BRIEF DESCRIPTION OF THE DRAWINGS

FIG. 1 is a schematic view showing a step (a) in a method of detecting a target ecDNA according to one embodiment.
FIG. 2 is a schematic view showing a step (b) in the method of detecting a target ecDNA according to one embodiment.
FIG. 3 is a schematic view showing a step (c) in the method of detecting a target ecDNA according to one embodiment.
FIG. 4 is a schematic view showing a step (c) in the method of detecting a target ecDNA according to one embodiment.
FIG. 5 is a view showing the positions of the designed primers and probes used in digital PCR in Experimental Example 1.
FIG. 6 is a diagram showing the classification of fluorescence of wells, where the fluorescence is detected after the digital PCR in Experimental Example 1.
FIG. 7 is a view showing the ratio of the target ecDNA copies to total copies of the MYC gene, which is measured in Experimental Example 1. The error bar indicates a standard error (N = 5). p < 0.05, t-test.
FIG. 8 is a view showing the ratio of circular plasmids, which is measured in a sample treated with SacI (SacI+) and a sample not treated with SacI (SacI-) from the circular plasmid DNA in Experimental Example 2. The error bar indicates a standard error (N = 3). p < 0.05, t-test.

### DETAILED DESCRIPTION OF THE INVENTION

Hereinafter, embodiments of the present invention will be described in detail with reference to the drawings as necessary. In the drawings, the same or corresponding parts are designated by the same or corresponding reference numerals, and the description thereof will not be duplicated. The dimensional ratio in each figure may be exaggerated for description and thus may not necessarily match the actual dimensional ratio.

### [Detection method for ecDNA]

A method of detecting a target ecDNA according to an embodiment is used for detecting a target ecDNA including a target nucleotide sequence in a sample containing DNA. The method of detecting a target ecDNA according to the embodiment includes (a) a step of subjecting the DNA in the sample to a treatment of sequence-specifically cleaving a third sequence present between a first sequence and a second sequence in the target nucleotide sequence; (b) a step of separating the sample after the step (a) into a plurality of compartments; and (c) a step of detecting a compartment containing DNA having both the first sequence and the second sequence in the plurality of compartments after the step (b). In the detection method for a target ecDNA according to the embodiment, the target ecDNA can be detected in a relatively short period of time and at a low cost without requiring complicated analysis and the like.

The "ecDNA" is a circular DNA derived from the chromosomal DNA, which is present in cells outside the chromosome. The "target ecDNA" refers to the ecDNA that is a detection target. The target ecDNA includes a target nucleotide sequence.

The "target nucleotide sequence" is a nucleotide sequence including a sequence serving as a detection target in the target ecDNA. The target nucleotide sequence is not particularly limited as long as it is a nucleotide sequence included in the ecDNA. Examples of the target nucleotide sequence include a nucleotide sequence included in a cancer gene. The term "cancer gene" is a gene that causes canceration of cells. Examples of the cancer gene include a gene of which the expression is specifically increased in cancer cells as compared with normal cells, and a gene in which a normal gene is mutated. Specific examples of the cancer gene include the MYC gene and the like; however, the cancer gene is not limited thereto.

In the method according to the embodiment, the sample serving as a detection target for the target ecDNA is a sample containing DNA. In one embodiment, the sample is a sample containing the chromosomal DNA and the ecDNA. Examples of the sample include a sample containing DNA derived from cells. Examples of the sample containing DNA derived from cells include a sample containing DNA extracted from cells. Extraction of the DNA from cells can be carried out using a publicly known method. The extraction of the DNA from cells may be carried out using a commercially available DNA extraction kit or the like.

### <Step (a)>

The step (a) is a step of subjecting DNA in a sample to a treatment of sequence-specifically cleaving a third sequence present between a first sequence and a second sequence in the target nucleotide sequence.

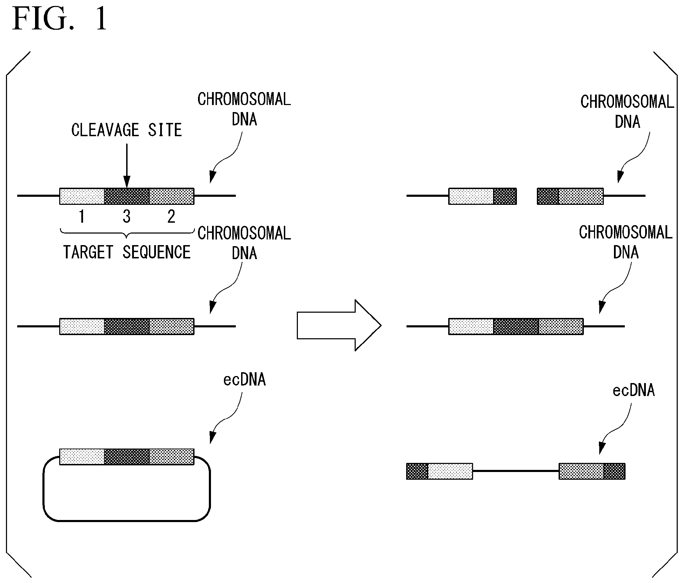

FIG. 1 is a schematic view showing an example of the step (a). The chromosomal DNA and the ecDNA are mixedly present in the sample. In the sample, the chromosomal DNA is considered to be present as a linear DNA, and the ecDNA is considered to be present as a circular DNA. The target nucleotide sequence (the "target sequence" in FIG. 1) is considered to be present in both the chromosomal DNA and the ecDNA. For this reason, even in a case where a target nucleotide sequence is detected in the sample, it is not possible to distinguish whether the target nucleotide sequence is derived from the chromosomal DNA or derived from the ecDNA.

The target nucleotide sequence includes a third sequence 3 between a first sequence 1 and a second sequence 2. In the step (a), the DNA in the sample is subjected to a treatment of cleaving the third sequence 3 in a sequence-specific manner. In a case where the chromosomal DNA including a target nucleotide sequence is cleaved at the third sequence 3, two molecules of linear DNAs, which are a DNA including the first sequence 1 and a part of the third sequence 3 and a DNA including the second sequence 2 and a part of the third sequence 3, are generated.

On the other hand, in a case where the target ecDNA is cleaved at the third sequence 3, one molecule of linear DNA, which includes a part of the first sequence 1 and the third sequence 3 at one terminal and includes a part of the second sequence 2 and the third sequence 3 at the other terminal, is generated.

Even in a case where the third sequence 3 is subjected to a cleaving treatment, the DNA that is not cleaved at the target nucleotide sequence may remain. In such a case, the target nucleotide sequence remains in a state where it includes the third sequence 3, between the first sequence 1 and the second sequence 2.

A publicly known method can be used for the treatment of cleaving the third sequence in a sequence-specific manner. Examples of the method for a cleaving treatment include a method using a sequence-specific nuclease. The term "sequence-specific nuclease" refers to an enzyme that recognizes a specific sequence and cleaves nucleic acid. Examples of the sequence-specific nuclease include a Cas protein in a system of Clustered Regularly Interspaced Short Palindromic Repeats (CRISPR)/Crisper Associated protein (Cas), a zinc-finger nuclease, and a transcription activator-like effector nuclease (TALEN). In one embodiment, the sequence-specific nuclease is a sequence-specific nuclease other than the restriction enzymes.

The sequence-specific nuclease may be an RNA-induced nuclease. The "RNA-induced nuclease" is an enzyme that forms a complex with RNA and then is induced by RNA to exhibit a sequence-specific nuclease activity. In the RNA-induced nuclease, RNA (hereinafter, also referred to as a "guide RNA") that forms a complex with an RNA-induced nuclease is complementarily bound to a complementary strand of a target sequence in the DNA. Next, the RNA-induced nuclease cleaves the DNA at the target sequence. As a result, it is possible to make the RNA-induced nuclease cleave DNA in any nucleotide sequence by changing the nucleotide sequence of the guide RNA.

Examples of the RNA-induced nuclease include the Cas protein. The Cas protein is a nuclease that is used in the CRISPR/Cas system. As the Cas protein, those known publicly can be used. Examples of the Cas protein include, but are not limited to, a Cas9 protein, a Cas12a (Cpf1) protein, a C2c1 protein, a C2c2 protein, a Cas3 protein, and a C2c3 protein.

The step (a) can be carried out by using a guide RNA and an RNA-induced nuclease that targets the third sequence. The guide RNA that targets the third sequence includes the target sequence in the third sequence as a guide sequence. In a case where the cleaving treatment for the third sequence is carried out with the CRISPR/Ca9 system, a sequence of about 15 to 30 nucleotides adjacent to the 5' side of the proto-spacer adjacent motif (PAM) sequence can be selected as the target sequence. The PAM varies depending on the kind of Cas protein to be used. The PAM corresponding to the Cas9 protein of S. pyogenes is "NGG". The PAM corresponding to the Cas9 protein of S. thermophilus is "NNAGAA". The PAM corresponding to the Cas9 protein of S. aureus is "NNGRRT" or "NNGRR(N)." The PAM corresponding to the Cas9 protein of N. meningitidis is "NNNNGATT". The PAM corresponding to the Cas9 protein of T. denticola is "NAAAAC" ("R" is A or G; "N" is A, T, G, or C).

The third sequence is not particularly limited as long as it is present between the first sequence and the second sequence. The first sequence and the second sequence are each a sequence serving as a detection target in the step (c) described later. In the step (c) described later, the length and position of each of the first sequence and the second sequence are not particularly limited as long as they are detectable. In the example of FIG. 1, the first sequence 1, the third sequence 3, and the second sequence 2 are adjacent to each other; however, the first sequence, the third sequence, and the second sequence do not need to be adjacent to each other. Any sequence may be interposed between the first sequence, the third sequence, and the second sequence.

After the cleaving treatment using the sequence-specific nuclease, a deactivation treatment for the sequence-specific nuclease may be carried out, and examples of the deactivation treatment method include a treatment with a proteolytic enzyme, a high temperature treatment, and the like.

### <Step (b)>

The step (b) is a step of separating the sample after the step (a) into a plurality of compartments.

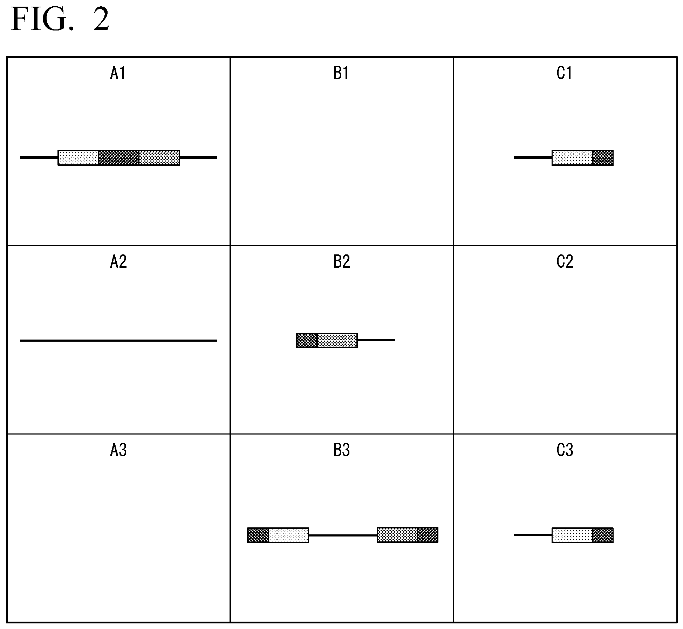

FIG. 2 is a schematic view showing an example of the step (b). In FIG. 2, one molecule of the DNA is distributed in each of compartments A1, A2, B2, B3, C1, and C3. The step (b) can be carried out so that, for example, one molecule or fewer of DNA is distributed in one compartment.

The step (b) can be carried out by diluting the sample after the step (a) and then distributing the diluted sample to a large number of micro-compartments. The compartment may be, for example, a micro-well or may be a micro-droplet. A method of distributing the sample to a compartment as a micro-droplet includes, for example, a method in which the sample after the step (a) is diluted with an aqueous solvent (for example, water), and the diluted aqueous solution is delivered into the oil phase.

A sample before being separated into a plurality of compartments can be allowed to have a DNA concentration such that one molecule or fewer of DNA is distributed to one compartment. The DNA concentration of the sample before being separated into a plurality of compartments is, for example, about 0.001 to 100 ng/µL; however, the DNA concentration thereof is not limited thereto.

A detection reagent for the first sequence, a detection reagent for the second sequence, a detection reagent for the third sequence, and the like may be added to the sample distributed to the plurality of compartments in order to carry out the step (c) described later. Examples of the detection reagent include a probe, a primer, a DNA polymerase, and dNTP (a mixture of dATP, dCTP, dGTP, and dTTP).

The step (b) may be carried out using a commercially available digital PCR device.

### <Step (c)>

The step (c) is a step of detecting a compartment containing DNA having both the first sequence and the second sequence in the plurality of compartments after the step (b).

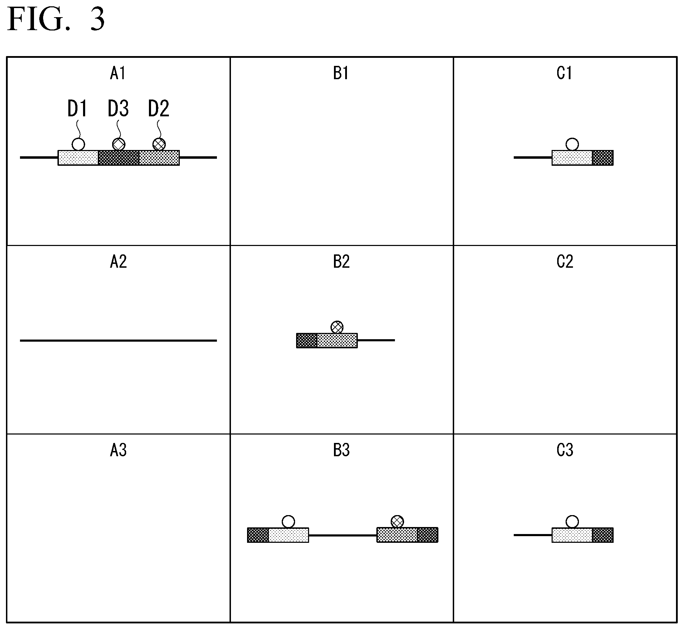

In the step (c), for example, a first fluorescent dye can be used to detect a compartment having the first sequence, and a second fluorescent dye can be used to detect a compartment having the second sequence. A third fluorescent dye can be used to detect a compartment having the third sequence. FIG. 3 is a schematic view showing an example of the step (c). In FIG. 3, the first sequence is detected by a first fluorescent dye D1. The second sequence is detected by a second fluorescent dye D2. The third sequence is detected by a third fluorescent dye D3. The fluorescent dyes D1 to D3 are each a fluorescent dye that generates fluorescence having a different wavelength.

The compartment A1 stores DNA that includes a target nucleotide sequence that has not been cleaved at the third sequence. Therefore, the fluorescent dyes D1 to D3 bind to the first sequence, the second sequence, and the third sequence, respectively, and the fluorescence generated from the mixing of the fluorescent dyes D1 to D3 is observed.

The compartment A2 stores DNA that does not include a target nucleotide sequence. Therefore, none of the fluorescence of the fluorescent dyes D1 to D3 is observed.

The compartment B2 stores DNA that is obtained by cleaving, at the third sequence, the chromosomal DNA including a target nucleotide sequence, where the DNA includes a part of the third sequence and the second sequence. Therefore, the fluorescence of the fluorescent dye D2 is observed.

The compartment B3 stores DNA that is obtained by cleaving the target ecDNA at the third sequence. Therefore, the fluorescence of each of the fluorescent dye D1 and the fluorescent dye D2 is observed.

The compartment C1 and the compartment C3 store DNA obtained by cleaving, at the third sequence, the chromosomal DNA including a target nucleotide sequence, where the DNA includes a part of the third sequence and the first sequence. Therefore, the fluorescence of the fluorescent dye D1 is observed.

As a result, in the example of FIG. 3, the compartment A1 and the compartment B3 are detected as compartments that includes DNA having both the first sequence and the second sequence.

In the example of FIG. 3, the compartment A1 stores DNA that includes a target nucleotide sequence that has not been cleaved at the third sequence. Therefore, the compartment A1 may be excluded as a compartment containing the target ecDNA. That is, in the step (c), a compartment containing DNA that has both the first sequence and the second sequence but does not have the third sequence which is uncleaved may be detected. In this case, in the example of FIG. 3, the fluorescence of each of the fluorescent dye D1 and the fluorescent dye D2 is observed, and the compartment B3 is detected as a compartment in which the fluorescence of the fluorescent dye D3 is not observed. The step (c) may include detecting a compartment in which the fluorescence of the fluorescent dye D1 for detecting the first sequence and the fluorescence due to the fluorescent dye D2 for detecting the second sequence are observed, but the fluorescence of the fluorescent dye D3 for detecting the third sequence is not observed.

In the step (c), compartments containing DNA that has both the first sequence and the second sequence but does not have the third sequence which is uncleaved may be detected, and the number of these compartments may be counted. Based on the number of the compartments, the target ecDNA can be detected. Alternatively, the target ecDNA in the sample can be quantified.

In each compartment, the first sequence, the second sequence, and the third sequence can be detected by a publicly known method. Examples of the publicly known method thereof include a method using a probe in which a fluorescent dye is bonded. For example, the detection of the first sequence can be carried out using a probe labeled with the fluorescent dye D1, which has a binding ability with respect to the first sequence. The detection of the second sequence can be carried out using a probe labeled with the fluorescent dye D2, which has a binding ability with respect to the second sequence. The detection of the third sequence can be carried out using a probe labeled with the fluorescent dye D3, which has a binding ability with respect to the third sequence. The probe having a binding ability with respect to the third sequence is designed so that the probe binds, for example, to a region including a cleavage site in the step (a).

The first sequence, the second sequence, and the third sequence may be detected by carrying out a nucleic acid amplification reaction. For example, the step (c) may include carrying out a nucleic acid amplification reaction for a region containing the first sequence and a nucleic acid amplification reaction for a region containing the second sequence. Alternatively, the step (c) may include carrying out a nucleic acid amplification reaction for a region containing the first sequence, a nucleic acid amplification reaction for a region containing the second sequence, and a nucleic acid amplification reaction for a region containing the third sequence.

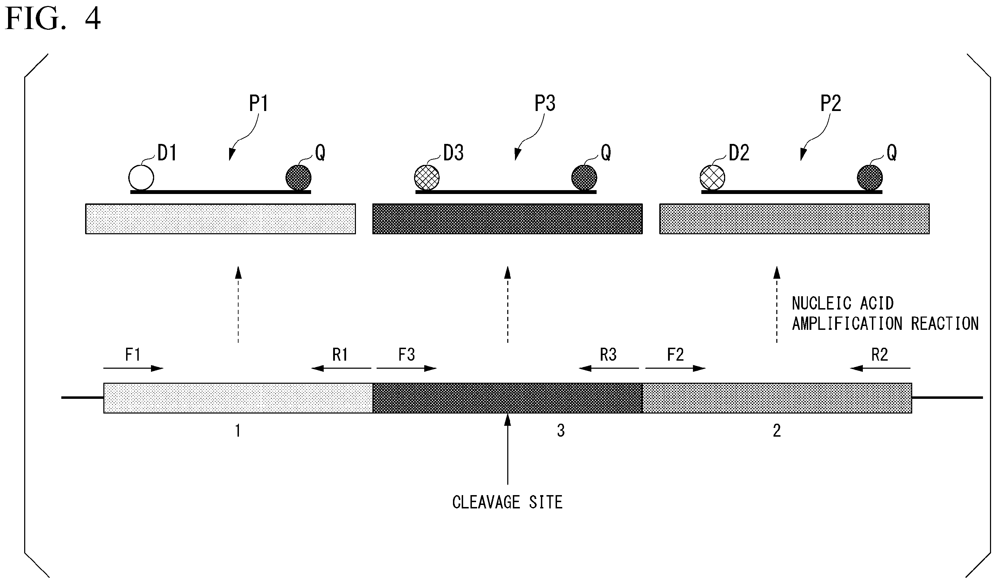

FIG. 4 is a schematic view showing an example in a case where a nucleic acid amplification reaction is carried out to detect the first sequence, the second sequence, and the third sequence. F1 and R1 are respectively a forward primer and a reverse primer for carrying out a nucleic acid amplification reaction of the first sequence 1. F2 and R2 are respectively a forward primer and a reverse primer for carrying out a nucleic acid amplification reaction of the second sequence 2. F3 and R3 are respectively a forward primer and a reverse primer for carrying out a nucleic acid amplification reaction of the third sequence 3. P1 is a probe having a binding ability with respect to the first sequence 1. P2 is a probe having a binding ability with respect to the second sequence 2. P3 is a probe having a binding ability with respect to the third sequence 3.

The primers F3 and R3 for the third sequence 3 are designed so that a fragment that is to be obtained by nucleic acid amplification includes a cleavage site in the step (a). In a case where the nucleic acid amplification reaction is carried out, the first sequence 1 and the second sequence 2 are amplified. In a case where the third sequence 3 has been cleaved in the step (a), the third sequence 3 is not amplified. On the other hand, in a case where the third sequence 3 has not been cleaved in the step (a), the third sequence 3 is amplified.

A nucleic acid amplification reaction can be carried out by a publicly known method. Examples of the nucleic acid amplification reactions include, but are not limited to, a polymerase chain reaction (PCR) method, a loop-mediated isothermal amplification (LAMP) method, a recombinase polymerase amplification (RPA) method, and a nicking enzyme amplification reaction (NEAR) method.

In a case where the nucleic acid amplification method is carried out by PCR, a probe obtained by bonding a fluorescent dye to a quencher may be used as the probe. In the example of FIG. 4, the fluorescent dyes D1 to D3 are respectively bonded to the respective 5' terminals of the probes P1 to P3, and quenchers Q are respectively bonded to the respective 3' terminals of the probes P1 to P3.

The probe P1 has a binding ability with respect to the first sequence 1, and it binds to the first sequence in a case where the first sequence 1 is present. In the probe P1, the fluorescent dye D1 and the quencher Q are present on the probe, and thus the generation of the fluorescence of the fluorescent dye D1 is suppressed by the quencher Q. In a case where a nucleic acid elongation reaction by a DNA polymerase occurs in a state where the probe P1 has bound to the first sequence 1, the probe P1 is degraded by the exonuclease activity possessed by the DNA polymerase. By this degradation, the fluorescent dye D1 is separated from the quencher Q. The fluorescence due to the fluorescent dye D1 is generated by separating the fluorescent dye D1 from the quencher Q. As the nucleic acid amplification reaction of the first sequence 1 proceeds, the amount of the fluorescent dye D1 separated from the quencher Q increases, and the intensity of the fluorescence due to the fluorescent dye D1 increases. For this reason, the fluorescence of the fluorescent dye D1 is generated in a compartment containing DNA including the first sequence. On the other hand, in a compartment that does not contain DNA including the first sequence, the fluorescence of the fluorescent dye D1 is not generated since the nucleic acid amplification reaction of the first sequence does not proceed.

The probe P2 has a binding ability with respect to the second sequence 2, and it binds to the second sequence in a case where the second sequence 2 is present. In the probe P2, the fluorescent dye D2 and the quencher Q are present on the probe, and thus the generation of the fluorescence of the fluorescent dye D2 is suppressed by the quencher Q. Regarding the probe P2 as well, in a similar manner as in the case of the probe P1, as the nucleic acid amplification reaction of the second sequence 2 proceeds, the amount of the fluorescent dye D2 separated from the quencher Q increases, and the intensity of the fluorescence due to the fluorescent dye D2 increases. For this reason, the fluorescence of the fluorescent dye D2 is generated in a compartment containing DNA including the second sequence. On the other hand, in a compartment that does not contain DNA including the second sequence, the fluorescence of the fluorescent dye D2 is not generated since the nucleic acid amplification reaction of the second sequence does not proceed.

The probe P3 has a binding ability with respect to the third sequence 3, and it binds to the third sequence in a case where the third sequence 3 is present. In the probe P3, the fluorescent dye D3 and the quencher Q are present on the probe, and thus the generation of the fluorescence of the fluorescent dye D3 is suppressed by the quencher Q. Regarding the probe P3 as well, in a similar manner as in the case of the probe P1, as the nucleic acid amplification reaction of the third sequence 3 proceeds, the amount of the fluorescent dye D3 separated from the quencher Q increases, and the intensity of the fluorescence due to the fluorescent dye D3 increases. In the step (a), in a case where the third sequence is cleaved, the nucleic acid amplification reaction of the third sequence does not proceed. For this reason, the fluorescence of the fluorescent dye D3 is not generated in a compartment containing DNA including the cleaved third sequence. The fluorescence of the fluorescent dye D3 is not generated even in a compartment that does not contain DNA including the third sequence. On the other hand, in a compartment containing DNA including the uncleaved third sequence, the fluorescence of the fluorescent dye D3 is generated since the nucleic acid amplification reaction of the third sequence proceeds.

A target ecDNA after the step (a) includes the first sequence, the second sequence, and the cleaved third sequence. Therefore, in the compartment containing a target ecDNA, the fluorescence of each of the fluorescent dye D1 and the fluorescent dye D2 is generated, but the fluorescence of the fluorescent dye D3 is not generated. As a result, the compartment in which the fluorescence of each of the fluorescent dye D1 and the fluorescent dye D2 is observed but the fluorescence of the fluorescent dye D3 is not observed can be detected as a compartment containing a target ecDNA.

Primers and probes can be designed by a publicly known method according to a method for a nucleic acid amplification reaction. The primer for the nucleic acid amplification reaction can include a forward primer and a reverse primer.

The lower limit value of the length of the primer is, for example, an oligomer of 15 mer or more, 16 mer or more, 17 mer or more, 18 mer or more, or 19 mer or more. The upper limit value of the length of the primer is, for example, an oligomer of 30 mer or less, 29 mer or less, 28 mer or less, 27 mer or less, 26 mer or less, or 25 mer or less. These upper limit values and lower limit values can be combined in any combination.

The lower limit value of the length of the probe is, for example, an oligomer of 15 mer or more, 16 mer or more, 17 mer or more, 18 mer or more, or 19 mer or more. The upper limit value of the length of the probe is, for example, an oligomer of 30 mer or less, 29 mer or less, 28 mer or less, 27 mer or less, 26 mer or less, or 25 mer or less. These upper limit values and lower limit values can be combined in any combination. It is preferable that the Tm value of the probe is higher than the Tm value of the primer by about 3°C to 10°C.

In a case where the probe includes a fluorescent dye and a quencher, the probe may be a double quencher probe. The double quencher probe includes a quencher at one position in the intermediate region of the probe in addition to any one of the 3' terminal or the 5' terminal.

For the fluorescent dye and the quencher, those publicly known can be used without any particular limitation. Examples of the fluorescent dye include; carboxyfluorescein (FAM), 6-carboxy-4', 5'-dichloro 2',7'-dimethoxyfluorescein (JOE), fluorescein isothiocyanate (FITC), tetrachlorofluorescein (TET), 5'-hexachloro-CE phosphoramideite (HEX), Cy3, Cy5, Alexa568, Alexa647, and the like. Examples of the quencher include TAMRA, ZEN, Iowa Black FQ, and the like.

A nucleic acid amplification reaction can be carried out by a publicly known method. In a case of a PCR method, for example, the nucleic acid amplification reaction can be carried out by repeating, after pre-denaturation, a cycle of denaturation, annealing, and elongation.

Examples of the pre-denaturation temperature include 94°C to 98°C, and 95°C is usually used. Examples of the time of pre-denaturation time include 20 seconds to 3 minutes.

Examples of the denaturation temperature include 94°C to 98°C, and 95°C is usually used. Examples of the denaturation time include 5 seconds to 30 seconds.

The annealing temperature can be set based on the Tm value of the primer. The annealing temperature can be set to, for example, a temperature lower than the Tm value of the primer by 2 to 3 degrees. Examples of the annealing temperature include 45°C to 65°C. Examples of the annealing time include 40 to 100 seconds.

Examples of the elongation temperature include 70°C to 75°C, and 70°C to 72°C is usually used. The elongation time can be set according to the length of the nucleic acid fragment to be amplified. Examples of the time of the elongation reaction include 30 to 100 seconds.

The annealing and the elongation may be carried out at the same time. In this case, examples of the temperature of annealing/elongation include 55°C to 65°C, and 60°C is usually used. The time of annealing/elongation can be set according to the length of the nucleic acid fragment to be amplified. Examples of the time of annealing/elongation include 30 to 100 seconds.

Examples of the number of cycles of denaturation, annealing and elongation include 30 to 100 cycles, and 40 to 60 cycles are preferable. Even in a case where the cycle is a cycle of denaturation and annealing/elongation, examples of the number of cycles include the same ones as those described above.

The step (c) includes, in the plurality of compartments after the step (b),
(i) carrying out a nucleic acid amplification reaction of the first sequence and detecting the first sequence based on the fluorescence of the fluorescent dye D1, by using a first primer set (F1 and R1) for carrying out the nucleic acid amplification reaction of the first sequence and a first probe P1 that has a binding ability with respect to a first nucleic acid fragment amplified by the nucleic acid amplification reaction with the first primer set and is labeled with the fluorescent dye D1;
(ii) carrying out a nucleic acid amplification reaction of the second sequence and detecting the second sequence based on the fluorescence of the fluorescent dye D2, by using a second primer set (F2 and R2) for carrying out the nucleic acid amplification reaction of the second sequence and a second probe P2 that has a binding ability with respect to a second nucleic acid fragment amplified by the nucleic acid amplification reaction with the second primer set and is labeled with the fluorescent dye D2; and
(iii) carrying out a nucleic acid amplification reaction of the third sequence and detecting the third sequence based on the fluorescence of the fluorescent dye D3, by using a third primer set (F3 and R3) for carrying out the nucleic acid amplification reaction of the third sequence and a third probe P3 that has a binding ability with respect to a third nucleic acid fragment amplified by the nucleic acid amplification reaction with the third primer set and is labeled with the fluorescent dye D3,
the step (c) may be a step of detecting the compartment in which the fluorescence of the fluorescent dye D1 and the fluorescence of the fluorescent dye D2 are observed but the fluorescence of the fluorescent dye D3 is not observed, as a compartment containing DNA having both the first sequence and the second sequence (hereinafter, also referred to as a "step (c')).

The step (b) and the step (c) can be carried out by a publicly known digital PCR method. The step (b) and the step (c) can be carried out, for example, by using a commercially available digital PCR device.

### <Other steps>

The method according to the embodiment may include other steps. Examples of the other steps include a step of eliminating a false positive compartment (hereinafter, also referred to as a "step (d)) and a step of calculating the ratio of the number of copies of the target ecDNA (hereinafter, also referred to as a "step (e)").

### (Step (d))

In the step (b), two molecules of DNAs, which are a DNA including the first sequence and a DNA including the second sequence, may be incidentally distributed to one compartment in a case where a sample is separated into a plurality of compartments. In such a compartment, in a case where the first sequence, the second sequence, and the third sequence are subjected to a detection operation, the results are such that the first sequence and the second sequence are detected, but the third sequence is not detected. For this reason, it cannot be distinguished from the compartment containing the target ecDNA. Such a compartment containing two molecules of DNAs as described above is false positive. The method according to the embodiment may include a step of eliminating this false positive compartment. More specifically, the step (d) may include a step of excluding a compartment containing at least two molecules of DNAs from the compartment detected in the step (c), where the two molecules of DNAs are a DNA having the first sequence and a DNA including the second sequence. Examples of the method of carrying out the step (d) include the following methods 1 to 3, and the step (d) may be carried out by any of methods of the methods 1 to 3.

### <<Method 1>>

The elimination of the false positive compartment may be carried out, for example, by using Expression (i) and Expression (v), which will be shown in examples described later. In Expression (i) and Expression (v), each symbol indicates the following meaning.
p, q, N(A), N(C), N(E), and N(U) indicate the following numbers obtained according to a method of carrying out the steps (a) to (c).
p: The number of compartments to which at least two molecules of DNAs are assumed to be distributed, where the DNAs are a DNA having the first sequence and a DNA having the second sequence.
q: The number of compartments containing the target ecDNA.
N(A): The number of compartments in which only the first sequence has been detected.
N(C): The number of compartments in which only the second sequence has been detected.
N(E): The number of compartments in which the first sequence and the second sequence have been detected but the third sequence has not been detected.
N(U): The number of all compartments in the step (b).

In Expression (i) and Expression (v), p indicates the number of compartments assumed to be false positive. By calculating p according to Expression (i) and subtracting q from N(E) according to Expression (v), the number q of compartments containing the target ecDNA can be obtained. In a case where the steps (a) and (b) are carried out by digital PCR, the distribution of DNA to each compartment is considered to follow a Poisson distribution. Therefore, the average number λ_{q} of copies of the target ecDNA per compartment can be calculated according to Expression (vi) shown in the examples below. The number Q of copies of the target ecDNA can be calculated according to Expression (vii) shown in the examples below.

### <<Method 2>>

After carrying out the step (a), the number of false positive compartments may be calculated by carrying out the step (b) using two or more kinds of samples having dilution rates different from each other. In a case where a sample of one-fold dilution and a sample of two-fold dilution are used, the number of compartments to which the target ecDNA is distributed in the case where the sample of two-fold dilution is used in the step (b) is 1/2 of the number of compartments in the case where the sample of one-fold dilution is used. On the other hand, in a case where a sample of two-fold dilution is used, the number of false positive compartments is 1/4 of the number of compartments in the case where the sample of one-fold dilution is used.

Based on the difference in the frequency of generation between the number of compartments of the target ecDNA and the number of false positive compartments in the case where the samples having dilution rates different from are used, the number of false positive compartments can be calculated.

### <<Method 3>>

The number of false positive compartments may be calculated before carrying out the step (b) by carrying out, in addition to the cleaving treatment in the step (a), a cleaving treatment (hereinafter, also referred to as a "cleaving treatment (z)" on a nucleotide sequence included in the target ecDNA, where the nucleotide sequence is different from the target nucleotide sequence. In the sample that has been subjected to the cleaving treatment of the step (a) and the cleaving treatment (z), the first sequence and the second sequence are separated into two DNA molecules in the target ecDNA. On the other hand, in the sample that has not been subjected to the cleaving treatment (z) but has been subjected to only the cleaving treatment of the step (a), the first sequence and the second sequence are not separated. As a result, the number N(E") of compartments in which the first sequence and the second sequence are detected in a case where the cleaving treatment (z) has been carried out can be regarded as false positive in a case where the cleaving treatment (z) is not carried out.

### (Step (e))

The ratio of the number of copies of the target ecDNA may be calculated, for example, according to any of the methods (I) to (III) shown in the examples below. In (I) to (III), the number of copies of the target DNA is calculated from the number of compartments in which the target DNA is present, based on the Poisson distribution. (I) to (III) can be applied, for example, in a case where the steps (b) and (c) are carried out by digital PCR.
(I) The calculation of the ratio of the number of copies of the target ecDNA to the total number of copies of the DNA including the target nucleotide sequence. The total number m of compartments in which the first sequence has been detected is calculated according to Expression (ii) shown in the examples.
   $m = N \left(A\right) + N \left(D\right) + N \left(E\right) + N \left(G\right)$
   N(D): The number of compartments in which the first sequence and the third sequence have been detected but the second sequence has not been detected
   N(E): The number of compartments in which the first sequence and the second sequence have been detected but the third sequence has not been detected
   N(G): The number of compartments in which the first sequence, the second sequence, and the third sequence have been detected

The average number λₘ of copies per compartment of the DNA including the first sequence is calculated according to Expression (iii) below.${λ}_{m} = - ln \left(1-\left(m/N\left(U\right)\right.\right)$

The number M of copies of the DNA including the first sequence is calculated according to Expression (vi) below. M can be used as the total number of copies of the DNA including the target nucleotide sequence.$M = {λ}_{m} \times N \left(U\right)$

The number q of wells containing the target ecDNA is calculated according to Expression (v) below.$q = N \left(E\right) - p$

The average number λ_{q} of copies of the target ecDNA per compartment is calculated according to Expression (vi) below.${λ}_{q} = - ln \left(1-\left(q/N\left(U\right)\right.\right)$

The number Q of copies of the target ecDNA is calculated according to Expression (vii) below.$Q = {λ}_{q} \times N \left(U\right)$

The proportion R of the number of copies of the target ecDNA to the total number M of copies of the DNA including the target nucleotide sequence is calculated according to Expression (viii) below.$R = Q / M$

In a case of considering a compartment containing a target nucleotide sequence that has not been cleaved by the step (a), by using the number of compartments that is obtained in a case where the step (a) has not been carried out, but the step (b) and the step (c) have been carried out (hereinafter, also referred to as a "sample not subjected to the step (a)"), the proportion R' of the number of copies of the target ecDNA to the total number M of copies of the DNA including the target nucleotide sequence in the sample not subjected to the step (a) is calculated according to Expressions (i') to (viii') shown in the examples described below. In a case of considering a compartment containing a target nucleotide sequence that has not been cleaved by the step (a), the proportion R¹ of the number of copies of the ecDNA to the total number M of copies of the DNA including the target nucleotide sequence can be calculated according to Expression (ix) below.${R}^{1} = R - R ′$

The symbols in Expressions (i') to (viii') described below have the following meanings.
p: The number of compartments to which at least two molecules of DNAs are assumed to be distributed, where the DNAs are a DNA having the first sequence and a DNA having the second sequence.
q: The number of compartments containing the target ecDNA.
N(A'): The number of compartments in which only the first sequence has been detected in the sample not subjected to the step (a).
N(C'): The number of compartments in which only the second sequence has been detected in the sample not subjected to the step (a).
N(E'): The number of compartments in which the first sequence and the second sequence have been detected but the third sequence has not been detected in the sample not subjected to the step (a).
N(U'): The number of all compartments in the step (b) in the sample not subjected to the step (a).
N(D'): The number of compartments in which the first sequence and the third sequence have been detected but the second sequence has not been detected in the sample not subjected to the step (a)
N(E'): The number of compartments in which the first sequence and the second sequence have been detected but the third sequence has not been detected in the sample not subjected to the step (a)
N(G'): The number of compartments in which the first sequence, the second sequence, and the third sequence have been detected in the sample not subjected to the step (a).
m': The total number of compartments in which the first sequence has been detected in the sample not subjected to the step (a)
λₘ': The average number of copies per compartment of the DNA including the first sequence in the sample not subjected to the step (a)
M': The total number of copies of the DNA including the target nucleotide sequence in the sample not subjected to the step (a)
q': The number of wells containing the target ecDNA in the sample not subjected to the step (a)
λ_{q}': The average number of copies per compartment of the target ecDNA in the sample not subjected to the step (a)
Q': The number of copies of the target ecDNA in the sample not subjected to the step (a)
R': The ratio of the number of copies of the target ecDNA to the total number M of copies of the DNA including the target nucleotide sequence in the sample not subjected to the step (a)

(II) The calculation of the ratio of the number of copies of the target ecDNA to the number of copies of the DNA cleaved by the step (a)

The number s of compartments in which the first sequence is detected but the third sequence is not detected is calculated according to Expression (x) below. s is considered to be the number of compartments containing DNA that includes the first sequence, where the DNA has been cleaved by the step (a).$s = N \left(A\right) + N \left(E\right)$

The average number λₛ of copies per compartment of the DNA including the first sequence, where the DNA has been cleaved by the step (a), is calculated according to Expression (xi) below.${λ}_{s} = - ln \left(1-\left(s/N\left(U\right)\right.\right)$

The number of copies S of the DNA including the first sequence, where the DNA has been cleaved by the step (a) is calculated according to Expression (xii) below.$S = {λ}_{s} \times N \left(U\right)$

The proportion R² of the number of copies of the target ecDNA to the number of copies of the DNA including the target nucleotide sequence cleaved by the step (a) is calculated according to Expression (xiii) below. ${R}^{2} = R / S$

(III) The calculation of the ratio of the number of copies of the target ecDNA to the number of copies of the DNA cleaved by the step (a), where a negative control which has not been subjected to the step (a) has been utilized for the calculation

The average number λ_{g} of copies per compartment of the DNA including all of the first sequence, the second sequence, and the third sequence is calculated according to Expression (xiv) below. For the sample not subjected to the step (a), the average number λ_{g}' of copies per compartment of the DNA including all of the first sequence, the second sequence, and the third sequence is calculated according to Expression (xiv') below.${λ}_{g} = - ln \left(1-\left(N\left(G\right)/N\left(U\right)\right.\right)$ ${λ}_{g} ′ = - ln \left(1-\left(N\left(G′\right)/N\left(U′\right)\right.\right)$

The number G of copies of the DNA including all of the first sequence, the second sequence, and the third sequence is calculated according to Expression (xv) below. For the sample not subjected to the step (a), the number G' of copies of the DNA including all of the first sequence, the second sequence, and the third sequence is calculated according to Expression (xv') below.$G = {λ}_{g} \times N \left(U\right)$ $G ′ = {λ}_{g} ′ \times N \left(U′\right)$

The proportion R³ of the number of copies of the target ecDNA to the number of copies of the DNA cleaved by the step (a) is calculated according to Expression (xvi) below.${R}^{3} = \left(Q/M-Q′/M′\right) / \left(G′/M′-G/M\right)$

According to the method according to the embodiment described above, the target ecDNA can be detected and quantified by a relatively simple method in a sample contaminated with the chromosomal DNA. In the method according to the embodiment, a next generation sequencer or the like is not used, and thus the method according to the embodiment can be carried out at a relatively low cost. In the method according to the embodiment, the cleaving treatment, the distribution to a plurality of compartments, and the detection of the first sequence and the second sequence can be carried out without requiring complicated operations. In the method according to the embodiment, the target ecDNA is detected in one molecule unit, and thus the target ecDNA can be detected with high sensitivity.

### [Detection kit for ecDNA]

A detection kit for a target ecDNA according to an embodiment includes (a) a cleaving reagent for sequence-specifically cleaving a third sequence present between a first sequence and a second sequence in the target nucleotide sequence, (b) a detection reagent for the first sequence, and (c) a detection reagent for the second sequence. By using the detection kit for the target ecDNA according to the embodiment, the target ecDNA can be detected relatively in a short period of time and at a low cost without requiring complicated analysis and the like.

### <Cleaving reagent (a)>

The cleaving reagent (a) is a cleaving reagent for sequence-specifically cleaving a third sequence present between a first sequence and a second sequence in the target nucleotide sequence.

Examples of the cleaving reagent include a sequence-specific nuclease that cleaves the third sequence in a sequence-specific manner. Examples of the sequence-specific nuclease include the same ones as those described above. Examples of the sequence-specific nuclease include a guide RNA and an RNA-induced nuclease, which target the third sequence. The RNA-induced nuclease is an RNA-type nuclease that is capable of forming a complex with the guide RNA. Examples of the RNA-induced nuclease include the same ones as those described above.

As the cleaving reagent, for example, a Cas protein of the CRISPR/Cas system and a guide RNA that targets the third sequence can be used. Examples of the Cas protein include the same ones as those described above, and for example, the Cas9 protein can be used.

### <Detection reagent (b) for first sequence>

Examples of the detection reagent for the first sequence include a probe having a binding ability with respect to the first sequence. As the probe, a probe labeled with a first fluorescent dye can be used. Examples of the first fluorescent dye include the same ones as those described above. The probe may be such that a quencher is bonded in addition to the first fluorescent dye. Examples of the probe having a binding ability with respect to the first sequence include the same ones as those described above.

As the probe, a nucleic acid including a sequence complementary to the first sequence can be used. The probe may be a DNA, may be an RNA, or may be a mixture of DNA and RNA.

The detection reagent for the first sequence may include a reagent for carrying out a nucleic acid amplification reaction for a region including the first sequence. Examples of such a reagent include a primer that is capable of undergoing specific annealing to a region including the first sequence. The primer can include a forward primer and a reverse primer. Examples of the primer for the first sequence include the same ones as those described above.

### <Detection reagent (c) for second sequence>

Examples of the detection reagent for the second sequence include a probe having a binding ability with respect to the second sequence. As the probe, a probe labeled with a second fluorescent dye can be used. Examples of the second fluorescent dye include the same ones as those described above. The second fluorescent dye is a fluorescent dye that generates fluorescence having a wavelength different from the wavelength of the first fluorescent dye. The probe may be such that a quencher is bonded in addition to the second fluorescent dye. Examples of the probe having a binding ability with respect to the second sequence include the same ones as those described above.

As the probe, a nucleic acid including a sequence complementary to the second sequence can be used. The probe may be a DNA, may be an RNA, or may be a mixture of DNA and RNA.

The detection reagent for the second sequence may include a reagent for carrying out a nucleic acid amplification reaction for a region including the second sequence. Examples of such a reagent include a primer that is capable of undergoing specific annealing to a region including the second sequence. The primer can include a forward primer and a reverse primer. Examples of the primer for the second sequence include the same ones as those described above.

### <Other Reagents>

The kit according to the embodiment may include other constituents in addition to those described above. Examples of the other constituents include (d) a detection reagent for the third sequence, a reagent for a nucleic acid amplification reaction, and the like.

### (Detection reagent (d) for third sequence)

Examples of the detection reagent for the third sequence include a probe having a binding ability with respect to the third sequence. The probe can include a cleavage site targeted by the cleaving reagent described in (a) described above. As the probe, a probe labeled with a third fluorescent dye can be used. Examples of the third fluorescent dye include the same ones as those described above. The third fluorescent dye is a fluorescent dye that generates fluorescence having a wavelength different from the wavelengths of the first fluorescent dye and the second fluorescent dye. The probe may be such that a quencher is bonded in addition to the third fluorescent dye. Examples of the probe having a binding ability with respect to the third sequence include the same ones as those described above.

As the probe, a nucleic acid including a sequence complementary to the third sequence can be used. The probe may be a DNA, may be an RNA, or may be a mixture of DNA and RNA.

The detection reagent for the third sequence may include a reagent for carrying out a nucleic acid amplification reaction for a region including the third sequence. Examples of such a reagent include a primer that is capable of undergoing specific annealing to a region including the second sequence. The primer can include a forward primer and a reverse primer. Examples of the primer for the third sequence include the same ones as those described above. The forward primer for the third sequence can include a sequence located on the 5' side of the cleavage site targeted by the cleaving reagent described in (a) described above. The reverse primer for the third sequence can include a sequence located on the 3' side of the cleavage site targeted by the cleaving reagent described in (a) described above. The nucleic acid fragment amplified by the primers for the third sequence includes a cleavage site targeted by the cleaving reagent described in (a) described above.

### (Reagent for nucleic acid amplification reaction)

The reagent for a nucleic acid amplification reaction can be appropriately selected according to a method for a nucleic acid amplification reaction. For example, in a case where the nucleic acid amplification reaction is carried out by PCR, the reagent for a nucleic acid amplification reaction can include a thermostable DNA polymerase (Taq DNA polymerase or the like), dNTP (a mixture of dATP, dCTP, dGTP, and dTTP), magnesium salt, and the like.

The kit according to the embodiment can be used in a method of detecting the above-described target ecDNA.

### [Examples]

The present invention will be described with reference to Examples; however, the present invention is not limited to Examples below.

### <Experimental Example 1: Detection of target ecDNA in cancer cells>

### [Detection of target ecDNA]

A target ecDNA was detected by using a MYC gene as a target nucleotide sequence and by using an ecDNA including the MYC gene as a target ecDNA. DNA samples extracted from HEK293 and COLO320DM were used. COLO320DM is a colon cancer cell line and is known to contain a lot of target ecDNA (ecDNA including MYC gene).

HEK293-derived DNA and COLO320DM-derived DNA, which had been purified by an open column method (NucleoBond HMW DNA, MACHEREY-NAGEL), were diluted with deionized exchange water so that the concentration thereof was 18 ng/µL. According to the manufacturer's protocol, 3 µL of the solution of the diluted DNA was mixed with 27 µL of a CRISPR/Cas9 reaction solution (Cas9 Nuclease, S. pyogenes, New England Biolabs). After the reaction, in order to deactivate Cas9, 1 µL of Proteinase K (QIAGEN) was added to 30 µL of the reaction solution, followed by a reaction at 37°C for 10 minutes. In order to deactivate Proteinase K, a reaction was further carried out at 95°C for 10 minutes. In the HEK293 sample, 1 µL of the reaction solution was used as a template for the digital PCR. In the COLO320DM sample, 1 µL of a sample that had been diluted 53 times in consideration of the increase in the number of copies of the MYC gene was used as a template for the digital PCR.

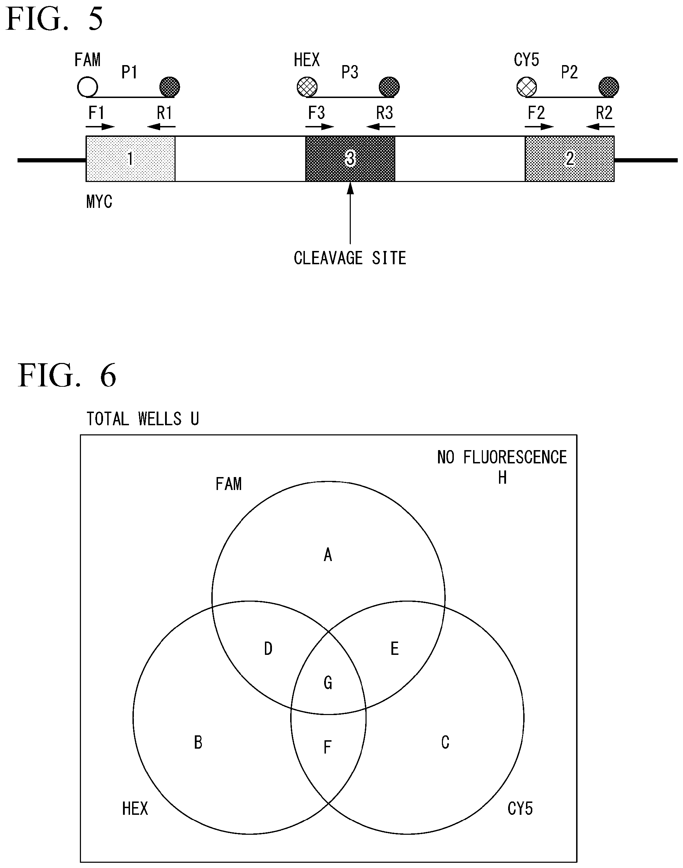

FIG. 5 is a schematic view showing the positions of the designed primers and probes which are used in the digital PCR. In the probe P1, FAM was used as a fluorescent dye. In the probe P2, CY5 was used as a fluorescent dye. In the probe P3, HEX was used as a fluorescent dye.

Table 1 shows the sequences of guide RNAs used in the cleaving reaction by CRISPR/Cas9, the sequences of the primers F1 to F3 and R1 to R3, and the sequences of the probes P1 to P3.

**[Table 1]**

| | | Sequence | Sequence number |
|---|---|---|---|
| Guide RNA | | CTTCGGGGAGACAACGACGG | 1 |
| Primer | F1 | CCGGTTTTCGGGGCTTTATC | 2 |
| | R1 | CCCTATTCGCTCCGGATCTC | 3 |
| | F2 | CAAGAGGCGAACACACAACG | 4 |
| | R2 | CAACTCCGGGATCTGGTCAC | 5 |
| | F3 | CTCCTACGTTGCGGTCACA | 6 |
| | R3 | TCCGGGTCGCAGATGAAAC | 7 |
| Probe | P1 | TGTAGTAATTCCAGCGAGAGGCAGAGGGA | 8 |
| | P2 | GTTTTAGCTCGTTCCTCCTCTGGCGCTC | 9 |
| | P3 | GTTCACCATGTCTCCTCCCAGCAGCTC | 10 |

Digital PCR was carried out using a QuantStudio Absolute Q Digital PCR System (Applied Biosystems). By the fluorescence of the respective fluorescent dyes, where P1 to P3 were used as the probes, each well exhibited fluorescence of any of the following A to H (see FIG. 6). The number of wells exhibiting fluorescence of each of A to H was individually counted.
A: Fluorescence of FAM alone
B: Fluorescence of HEX alone
C: Fluorescence of CY5 alone
D: Fluorescence of FAM and HEX
E: Fluorescence of FAM and CY5
F: Fluorescence of HEX and CY5
G: Fluorescence of FAM, HEX, and CY5
H: No fluorescence

### [Calculation of ratio of ecDNAs]

A well containing the target ecDNA exhibits fluorescence of E. However, even in a case where two molecules of DNAs, which are a DNA including the first sequence and a DNA including the second sequence, are distributed to one well by chance, the well also exhibits fluorescence of E. Therefore, in order to exclude such a false positive well, any of the following methods (I) to (III) can be employed.
(I) The calculation of the ratio of the number of copies of the target ecDNA to the total number of copies of the MYC gene
   (1) Digital PCR was carried out in the same way as described above, except that the cleavage by CRISPR/Cas9 was not carried out. The number of wells exhibiting fluorescence of each of A to H was individually counted. In (2) to (6) below, each fluorescence by this digital PCR was indicated as A' to H'.
   (2) By using two molecules of DNAs, which are a DNA detected by a probe 1 and a DNA detected by a probe 2, the number p of wells exhibiting fluorescence of E was calculated according to the following quadratic equation (i). For the results of the digital PCR in (1) described above, the number p of wells was also calculated in the same manner according to the following quadratic equation (i').
      [Equation 1] $\begin{matrix}\frac{\left\{p+N\left(A\right)\right\} \left\{p+N\left(C\right)\right\}}{N \left(U\right)} = p \left(i\right) \\ \frac{\left\{p′+N\left(A′\right)\right\} \left\{p′+N\left(C′\right)\right\}}{N \left(U′\right)} = p ′ \left(i'\right)\end{matrix}$
      N(A): The number of wells exhibiting fluorescence of A
      N(C): The number of wells exhibiting fluorescence of C
      p: The number of wells exhibiting fluorescence of E by two molecules of DNAs N(U): The number of all wells
      N(A'): The number of wells exhibiting fluorescence of A in the digital PCR of (1)
      N(C'): The number of wells exhibiting fluorescence of C in the digital PCR of (1)
      p': The number of wells exhibiting fluorescence of E by two molecules of DNAs in the digital PCR of (1)
      N(U'): The number of all wells in the digital PCR of (1)
   (3) The total number m of wells that exhibited the fluorescence of FAM was calculated according to Expression (ii) below. For the results of the digital PCR in (1) described above, the total number m' of wells that exhibited the fluorescence of FAM was also calculated in the same manner according to Expression (ii') below.
      $m = N \left(A\right) + N \left(D\right) + N \left(E\right) + N \left(G\right)$ $m ′ = N \left(A′\right) + N \left(D′\right) + N \left(E′\right) + N \left(G′\right)$
      N(D): The number of wells that exhibit fluorescence of D
      N(E): The number of wells that exhibit fluorescence of E
      N(G): The number of wells that exhibit fluorescence of G
      N(D'): The number of wells that exhibit fluorescence of D in the digital PCR of (1)
      N(E'): The number of wells that exhibit fluorescence of E in the digital PCR of (1)
      N(G'): The number of wells that exhibit fluorescence of G in the digital PCR of (1)

The average number λₘ of copies of the DNA detected with a probe 1 per well was calculated according to Expression (iii) below based on the Poisson distribution. For the results of the digital PCR in (1) described above, the average number λₘ' of copies of the DNA detected with the probe 1 per well was also calculated in the same manner according to Expression (iii') below.${λ}_{m} = - ln \left(1-\left(m/N\left(U\right)\right)\right.$ ${λ}_{m} ′ = - ln \left(1-\left(m′/N\left(U′\right)\right)\right.$

The number M of copies of the DNA detected with the probe 1 was calculated according to Expression (vi) below. For the results of the digital PCR in (1) described above, the number M' of copies of the DNA detected with the probe 1 was also calculated in the same manner according to Expression (vi') below.$M = {λ}_{m} \times N \left(U\right)$ $M ′ = {λ}_{m} ′ \times N \left(U′\right)$

M was defined as the total number of copies of the MYC gene. M' was defined as the total number of copies of the MYC gene in (1) described above.

(4) The number q of wells containing the target ecDNA was calculated by subtracting p from the number of wells exhibiting fluorescence of E according to Expression (v) below. For the results of the digital PCR in (1) described above, the number q' of wells containing the target ecDNA was also calculated in the same manner according to Expression (v') below.$q = N \left(E\right) - p$ $q ′ = N \left(E′\right) - p ′$
N(E): The number of wells exhibiting fluorescence of E
q': The number of wells containing the target ecDNA
N(E'): The number of wells exhibiting fluorescence of E in the digital PCR of (1)
q': The number of wells containing the target ecDNA in the digital PCR of (1)

The average number λ_{q} of copies of the target ecDNA per well was calculated according to Expression (vi) below based on the Poisson distribution. For the results of the digital PCR in (1) described above, the average number λ_{q}' of copies of the target ecDNA per well was also calculated in the same manner according to Expression (vi') below.${λ}_{q} = - ln \left(1-\left(q/N\left(U\right)\right)\right.$ ${λ}_{q} ′ = - ln \left(1-\left(q′/N\left(U′\right)\right.\right)$

The number Q of copies of the target ecDNA was calculated according to

Expression (vii) below. For the results of the digital PCR in (1) described above, the number Q' of copies of the target ecDNA was also calculated in the same manner according to Expression (vii') below.$Q = {λ}_{q} \times N \left(U\right)$ $Q ′ = {λ}_{q} ′ \times N \left(U′\right)$

The ratio R of the number of copies of the target ecDNA to the total number M of copies of the MYC gene was calculated according to Expression (viii) below. For the results of the digital PCR in (1) described above, the ratio R' of the number of copies of the target ecDNA to the total number M of copies of the MYC gene was also calculated in the same manner according to Expression (viii') below.$R = Q / M$ $R ′ = Q ′ / M ′$

R' is the ratio of the target ecDNA, which is calculated in a negative control in which the cleavage reaction by CRISPR/Cas9 is not carried out, and it is considered to be noise. In a case where this noise is eliminated, the ratio R¹ of the number of copies of ecDNA to the total number M of copies of the MYC gene can be calculated according to Expression (ix) below.${R}^{1} = R - R ′$

(II) The calculation of the ratio of the number of copies of the target ecDNA to the number of copies of the MYC gene cleaved by CRISPR/Cas9

The number s of wells that exhibited the fluorescence of FAM but did not exhibit the fluorescence of HEX was calculated according to Expression (x) below. s is considered to be the number of wells containing DNA detected with the probe 1, where the DNA has been cleaved by CRISPR/Cas9.$s = N \left(A\right) + N \left(E\right)$

The average number λₛ of copies per well of the DNA detected with the probe 1, where the DNA has been cleaved by CRISPR/Cas9, was calculated according to Expression (xi) below based on the Poisson distribution.${λ}_{s} = - ln \left(1-\left(s/N\left(U\right)\right.\right)$

The number S of copies of the DNA detected with the probe 1, where the DNA has been cleaved by CRISPR/Cas9, was calculated according to Expression (xii) below.$S = {λ}_{s} \times N \left(U\right)$

The ratio R² of the number of copies of the target ecDNA to the number of copies of the MYC gene cleaved by CRISPR/Cas9 was calculated according to Expression (xiii) below.${R}^{2} = R / S$

(III) The calculation of the ratio of the number of copies of the target ecDNA to the number of copies of the MYC gene cleaved by CRISPR/Cas9, where a negative control which has not been subjected to the cleavage reaction by CRISPR/Cas9 has been utilized for the calculation

The average number λ_{g} of copies of the DNA detected with all of the probe 1, the probe 2, and the probe 3 per well was calculated according to Expression (xiv) below based on the Poisson distribution. For the results of the digital PCR in (1) described above, the average number λ_{g}' of copies of the DNA detected with all of the probe 1, the probe 2, and the probe 3 per well was also calculated in the same manner according to Expression (xiv') below.${λ}_{g} = - ln \left(1-\left(N\left(G\right)/N\left(U\right)\right.\right)$ ${λ}_{g} ′ = - ln \left(1-\left(N\left(G′\right)/N\left(U′\right)\right.\right)$

The number of G of copies of the DNA detected with all of the probe 1, the probe 2, and the probe 3 was calculated according to Expression (xv) below. For the results of the digital PCR in (1) described above, the number G' of copies of the DNA detected with all of the probe 1, the probe 2, and the probe 3 per well was also calculated in the same manner according to Expression (xv') below.$G = {λ}_{g} \times N \left(U\right)$ $G ′ = {λ}_{g} ′ \times N \left(U′\right)$

The ratio R³ of the number of copies of the target ecDNA to the number of copies of the MYC gene cleaved by CRISPR/Cas9 was calculated according to Expression (xvi) below.

### [Results]

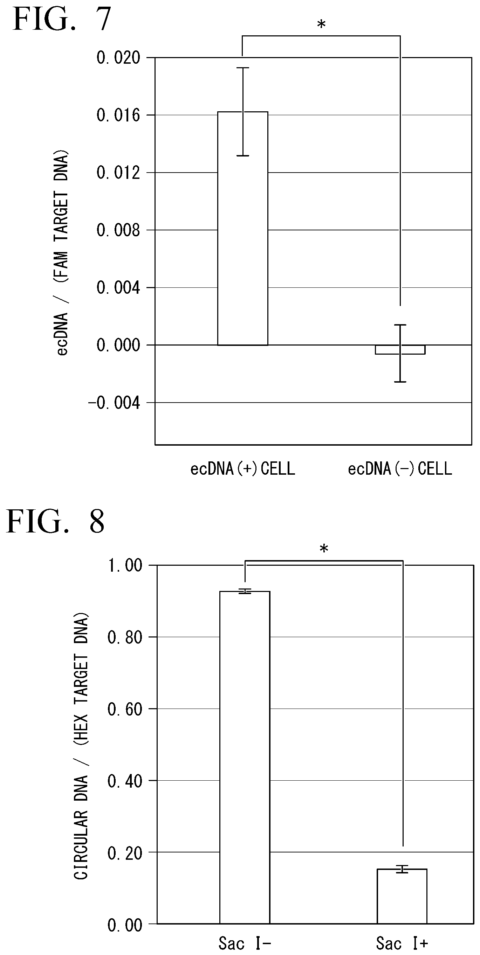

The measurement results of the target ecDNA are shown in FIG. 7. FIG. 7 shows the ratio of the target ecDNA, which has been calculated according to Expression (viii) described above. "ecDNA (+) cell" indicates the ratio of target ecDNAs in the COLO320DM-derived DNA. "ecDNA (-) cell" indicates the ratio of target ecDNAs in the sample of the HEK293-derived DNA. It has been confirmed that in the COLO320DM-derived DNA, a lot of the target ecDNA (the ecDNA including the MYC gene) is contained as compared with the HEK293-derived DNA

From this result, it has been confirmed that the number and ratio of target ecDNAs can be measured by the above-described method in DNA sample in which the chromosomal DNA and the ecDNA are mixedly present.

### <Experimental Example 2: Verification using circular plasmid DNA>

In an experimental system using a circular plasmid DNA, it was verified whether the circular DNA could be measured by the method described above. Cancer cells used in Experimental Example 1 contain a large number of components other than the DNA. Therefore, it was verified whether a circular DNA and a non-circular DNA could be distinguished by the above method in an experimental system containing only DNA.

A circular plasmid containing the MYC gene was used as the circular DNA. As the non-circular DNA, a sample obtained by treating the circular plasmid with a restriction enzyme SacI was used. The circular plasmid has only one SacI recognition sequence at a site other than the sequence of the MYC gene. Therefore, in a case where the circular plasmid is treated with SacI, the SacI recognition sequence present at a site other than the sequence of the MYC gene is cleaved, which results in a non-circular DNA containing the MYC gene. Hereinafter, a sample in which the circular plasmid has been treated with SacI is denoted as a sample (SacI+). A circular plasmid which has not been treated with SacI is denoted as a sample (SacI-).

The sample (SacI-) and the sample (SacI+) were each treated with a restriction enzyme PteI. After each sample was subjected to the deactivation treatment of PteI, digital PCR was carried out using primers F2 and R2, and a primer P2, as well as primers F3 and R3, and a probe P3, which were shown in Table 1. In the MYC gene shown in FIG. 5, the PteI recognition sequence is located between a region 3 and a region 2.

For each sample, the number of wells that exhibited the fluorescence of each fluorescent dye was counted after digital PCR. By the same method as in [Detection of target ecDNA] described above, the ratio of the number of the circular DNAs to the total number of copies of the MYC gene (HEX target DNA) in the sample (SacI+) and the sample (SacI-) was calculated.

The results are shown in FIG. 8. FIG. 8 shows the ratio of the circular DNAs, which has been calculated according to Expression (viii) described above. In the sample (SacI-), the ratio of the number of the circular DNAs to the total number of copies of the MYC gene was close to 1. On the other hand, in the sample (SacI+), the ratio of the number of the circular DNAs to the total number of copies of the MYC gene was less than 0.2. From these results, it has been confirmed that the circular DNA and the non-circular DNA can be distinguished by the above method.

Some embodiments have been described. However, these embodiments are presented as examples and thus are not intended to limit the scope of the invention. These embodiments can be carried out in various other forms, and various omissions, replacements, and changes can be made to the extent that they do not deviate from the gist of the invention. These embodiments and the modification thereof are included in the scope and gist of the invention and also included in the scope of the invention described in the present patent claims and the scope of its equivalents. While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.
1 First sequence
2 Second sequence
3 Third sequence
P1 First probe
P2 Second probe
P3 Third probe
F1 First forward primer
F2 Second forward primer
F4 Third forward primer
R1 First reverse primer
R2 Second reverse primer
R3 Third reverse primer
D1 First fluorescent dye
D2 Second fluorescent dye
D3 Third fluorescent dye

[Sequence Listing]

## Claims

1. A method of detecting a target ecDNA in a sample containing DNA, the method comprising:
(a) a step of subjecting the DNA in the sample to a treatment of sequence-specifically cleaving a third sequence present between a first sequence and a second sequence in a target nucleotide sequence included in the target ecDNA;
(b) a step of separating the sample after the step (a) into a plurality of compartments; and
(c) a step of detecting a compartment containing DNA having both the first sequence and the second sequence in the plurality of compartments after the step (b).

2. The method according to claim 1,
wherein the step (a) is carried out by using a guide RNA and an RNA-induced nuclease that targets the third sequence.

3. The method according to claim 1 or 2,
wherein the step (c) includes carrying out a nucleic acid amplification reaction for a region containing the first sequence and a nucleic acid amplification reaction for a region containing the second sequence.

4. The method according to any one of claims 1 to 3,
wherein in the step (c), a compartment containing DNA that has both the first sequence and the second sequence but does not have the third sequence which is uncleaved is detected.

5. The method according to claim 4,
wherein the step (c) includes carrying out a nucleic acid amplification reaction for a region containing the first sequence, a nucleic acid amplification reaction for a region containing the second sequence, and a nucleic acid amplification reaction for a region containing the third sequence.

6. The method according to any one of claims 1 to 5,
wherein the step (b) is carried out so that one molecule or fewer of DNA is distributed in one compartment.

7. The method according to any one of claims 1 to 6,
wherein the target nucleotide sequence is a nucleotide sequence included in a cancer gene.

8. The method according to any one of claims 1 to 7,
wherein the step (b) and the step (c) are carried out by digital PCR.

9. The method according to any one of claims 1 to 8,
wherein in the step (c), a first fluorescent dye is used to detect a compartment having the first sequence, and a second fluorescent dye is used to detect a compartment having the second sequence.

10. The method according to any one of claims 1 to 9,
wherein in the step (c), a third fluorescent dye is used to detect a compartment having the third sequence.

11. The method according to any one of claims 1 to 10, further comprising:
(d) a step of excluding a compartment containing at least two molecules of DNAs from the compartment detected in the step (c), where the two molecules of DNAs are a DNA including the first sequence and a DNA including the second sequence.

12. The method according to Claim 11,
wherein the step (d) is carried out using the following numerical expressions (i) and (ii), $\frac{\left\{p+N\left(A\right)\right\} \left\{p+N\left(C\right)\right\}}{N \left(U\right)} = p$ $q = N \left(E\right) - p$
wherein p is the number of compartments which are assumed to contain at least two molecules of DNAs, which are a DNA having the first sequence and a DNA having the second sequence, N(A) is the number of compartments in which only the first sequence is detected, N(C) is the number of compartments in which only the second sequence is detected, N(U) is the number of all compartments in the step (b), q is the number of compartments containing the target ecDNA, and N(E) is the number of compartments in which the first sequence and the second sequence have been detected but the third sequence has not been detected.

13. A kit for detecting a target ecDNA including a target nucleotide sequence in a sample containing DNA, the kit comprising:
(a) a cleaving reagent for sequence-specifically cleaving a third sequence present between a first sequence and a second sequence in the target nucleotide sequence;
(b) a detection reagent for the first sequence; and
(c) a detection reagent for the second sequence.

14. The kit according to claim 13,
wherein the cleaving reagent contains a guide RNA and an RNA-induced nuclease that targets the third sequence.

15. The kit according to claim 13 or 14, further comprising:
(d) a detection reagent for the third sequence.
